# EUROPEAN PATENT APPLICATION

(11) **EP 4 450 131 A1**
(43) Date of publication of application: **23.10.2024**
(21) Application number: 23169109.8
(22) Date of filing: 21.04.2023
(51) Int. Cl.: A61Q 19/00, A61K 8/92

(54) **MASSAGE OIL**

(71) Applicant: Medela Holding AG, 6340 Baar (CH)
(72) Inventor: MIOTTO, Letizia, 8055 Zurich (CH); AMBROSECCHIA, Paola, 8044 Zurich (CH); OHIOZEUA, Jeremiah, 6330 Cham (CH); YANG, Wei, Toronto, M1B 5N4 (CA)
(74) Representative: Grünecker Patent- und Rechtsanwälte PartG mbB

(57) **Abstract**

The present invention relates to a composition comprising *Persea gratissima* oil, *Olea europaea* oil, *Calendula officinalis* extract and *Zingiber officinale* oil and to the use of such a composition for providing warming relief to sore breasts.

## Description

### Field of the Invention

The present invention relates to a composition comprising *Persea gratissima* oil, *Olea europaea* oil, *Calendula officinalis* extract and *Zingiber officinale* oil. The present invention also relates to the use of such a composition for providing warming relief to sore breasts.

### Background of the Invention

Starting at the very beginning of and continuing throughout pregnancy, the female body is preparing to breastfeed. Triggered by a change in hormone levels (such as estrogen, progesterone and prolactin), many bodily changes take place in a short amount of time: blood flow to the breasts increases, fresh layers of fat begin to build, and the milk ducts start to expand, resulting in larger and more tender breasts.

Breastfeeding mothers commonly experience a feeling of tightness in the breast. The discomfort may go away once breastfeeding is started regularly but quite often the tightness sensation returns shortly after every breastfeeding session. In some cases, application of heat (e.g. applying a warm washcloth to the areolas at the start of each nursing session) can help; in other cases cold provides relief. In combination with temperature, (self-) massaging the breast can also provide relief. The massage can be further assisted by the use of a cream or an oil.

There still remains a need for products which can be used for massaging and relieving sore breasts.

### Summary of the Invention

Unexpectedly, it has been found that a composition comprising *Persea gratissima* oil, *Olea europaea* oil, *Calendula officinalis* extract and *Zingiber officinale* oil provides benefits such as nourishing dry skin and/or a warming sensation to the skin of breastfeeding women.

In a first aspect, the present invention relates to a composition comprising *Persea gratissima* oil, *Olea europaea* oil, *Calendula officinalis* extract and *Zingiber officinale* oil.

In a further aspect, the present invention relates to the cosmetic use of the composition in relieving sore breasts.

### Detailed description of the invention

The present invention relates to a composition comprising *Persea gratissima* oil, *Olea europaea* oil, *Calendula officinalis* extract and *Zingiber officinale* oil.

As will be discussed in greater detail below, the composition according to the invention provides a number of unique advantages in naturally relieving, soothing and calming the breast when used by breast-feeding or breast milk-pumping women. The inventors have found that the ingredients specified above and explained in greater detail below combine the advantages of a composition with especially lightweight viscosity for easy applicability to sore areas of the body, e.g. the breast, a natural origin which obviates concerns of ingestion and which allows use as a "leave-on product" which need not be removed between breast-feeding or breast milk-pumping sessions, while conditioning and soothing the area to which the inventive composition is applied. Further embodiments and advantages of the invention are discussed below.

In a preferred embodiment in optional combination with any of the above or below aspects or embodiments, the *Olea europaea* oil is *Olea europaea* fruit oil (such as for example designated by CAS (Chemical Abstracts Services) registry number 8001-25-0), and/or the *Calendula officinalis* extract is *Calendula officinalis* flower extract (such as for example designated by CAS registry number 84776-23-8), and/or the *Zingiber officinale* oil is *Zingiber officinale* root oil (such as for example designated by CAS registry number 8007-08-7). In a further preferred embodiment in optional combination with any of the above or below aspects or embodiments, the *Olea europaea* oil is *Olea europaea* fruit oil, the *Calendula officinalis* extract is *Calendula officinalis* flower extract and the *Zingiber officinale* oil is *Zingiber officinale* root oil.

The *Persea gratissima* oil herein may for example be designated by CAS registry number 8024-32-6).

In an embodiment in optional combination with any of the above or below aspects or embodiments, the composition comprises about 80% to about 100% by weight, preferably about 85% to about 99% by weight, more preferably about 90% to about 96% by weight, even more preferably about 91% to about 92 % by weight, most preferably about 91.9% by weight of the *Persea gratissima* oil.

In an embodiment in optional combination with any of the above or below aspects or embodiments, the composition comprises about 1% to about 20% by weight, preferably about 3% to about 15% by weight, more preferably about 5% to about 10% by weight, most preferably about 7% by weight of the *Olea europaea* oil.

In an embodiment in optional combination with any of the above or below aspects or embodiments, the composition comprises about 0.01% to about 5% by weight, preferably about 0.1 % to about 3% by weight, more preferably about 0.5% to about 2.5% by weight, most preferably about 1 % by weight of the *Calendula officinalis* extract.

In an embodiment in optional combination with any of the above or below aspects or embodiments, the composition comprises about 0.001% to about 2% by weight, preferably about 0.01 % to about 1% by weight, more preferably about 0.05% to about 0.5% by weight, even more preferably about 0.095% by weight to about 0.15 % by weight, most preferably about 0.1 % by weight of the *Zingiber officinale* oil.

In an embodiment in optional combination with any of the above or below aspects or embodiments, the cumulative amount of *Persea gratissima* oil, *Olea europaea* oil, *Calendula officinalis* extract and *Zingiber officinale* oil in the composition is more than about 90% by weight, preferably more than about 95% by weight, more preferably more than about 99% by weight.

In a preferred optional embodiment in combination with any of the above or below aspects or embodiments, the composition essentially consists of *Persea gratissima* oil, *Olea europaea* oil, *Calendula officinalis* extract and *Zingiber officinale* oil.

In an embodiment in optional combination with any of the above or below aspects or embodiments, the ingredients are of certified organic origin , for example Cosmos (COSMetic Organic and natural Standard) certification, e.g. Ecocert certification.

In an embodiment in optional combination with any of the above or below aspects or embodiments, the inventive composition is in the form of an oil.

In a further aspect, the present invention relates to the cosmetic use of the composition for relieving sore breasts and/or dry skin.

Technical and scientific terms used hereafter carry the commonly understood meaning of the word unless defined otherwise.

As used herein, each of the terms "comprising', "having" and "containing", including grammatical variants thereof, are meant in a non-exhaustive sense to mean "including", but not necessarily "composed of", and does not exclude elements in addition to those explicitly recited as being present. As used herein, then, the terms "comprising", "having" and "containing", and grammatical variants thereof, indicate that components other than those explicitly recited may, but need not, be present. As such these terms include as a limiting case embodiments in which no other elements than those recited are present, e.g. in the commonly accepted sense of "consisting of".

As used herein, the phrase "consisting of", and grammatically related variants thereof, means that no other elements are present in those recited. In standing with the above definition of "comprising" (and grammatically and semantically related terms), the term "consisting of' therefore denotes a limiting scenario within the meaning of "comprising".

Unless defined otherwise, any feature within any aspect or embodiment of the invention may be combined with any feature within any other aspect or embodiment of the invention, and the skilled person understands such combination as being encompassed in the original disclosure of the present application. This applies in particular to all embodiments described within the section relating to the composition *per se,* in respect of other aspects, e.g. methods and uses, of those compositions. This also applies in particular, but not exclusively, to endpoints of ranges disclosed herein. For instance, if a given substance is disclosed as existing in a composition in a concentration range of about X-Y% or about A-B%, the present application is to be understood as explicitly disclosing not only the ranges about X-Y% and about A-B%, but also the ranges about X-B%, about A-Y% and, in as far as numerically possible, about Y-A% and about B X%. Each of these ranges, and range combinations, are contemplated, and are to be understood as being directly and unambiguously disclosed in the present application.

Unless stated otherwise, the designation of a range in the present application using a hyphen ("-") separating two bracketing values X and Y, or two bracketing ratios, is to be understood as meaning and disclosing the specified range in which both endpoint values X and Y are included. The same applies to a range expressed as "from about X to about Y". Accordingly, the expressions of ranges as "X -Y", "of X to Y", "from X to Y", "of X - Y" and "from X - Y" are to be understood equivalently as meaning and disclosing a range encompassing the end value X, all values between X and Y, as well as the end value Y.

The designation of a range in the present application using the word "between" preceding two bracketing values X and Y, or two bracketing ratios, is to be understood as meaning and disclosing the specified range in which both endpoint values X and Y are excluded, but all values between the specified endpoint values X and Y are included. As used herein the term "about" when referring to a particular value, e.g. an endpoint or endpoints of a range, encompasses and discloses, in addition to the specifically recited value itself, a certain variation around the specifically recited value. Such a variation may for example arise from normal measurement variability, e.g. in the weighing or apportioning of various substances by methods known to the skilled person. The term "about" shall be understood as encompassing and disclosing a range of variability above and below an indicated specific value, said percentage values being relative to the specific recited value itself, as follows. The term "about" may denote variability of ± 5.0%. The term "about" may denote variability of ± 4.9%. The term "about" may denote variability of ± 4.8%. The term "about" may denote variability of ± 4.7%. The term "about" may denote variability of ± 4.6%. The term "about" may denote variability of ± 4.5%. The term "about" may denote variability of ± 4.4%. The term "about" may denote variability of ± 4.3%. The term "about" may denote variability of ± 4.2%. The term "about" may denote variability of ± 4.1%. The term "about" may denote variability of ± 4.0%. The term "about" may denote variability of ± 3.9%. The term "about" may denote variability of ± 3.8%. The term "about" may denote variability of ± 3.7%. The term "about" may denote variability of ± 3.6%. The term "about" may denote variability of ± 3.5%. The term "about" may denote variability of ± 3.4%. The term "about" may denote variability of ± 3.3%. The term "about" may denote variability of ± 3.2%. The term "about" may denote variability of ± 3.1%. The term "about" may denote variability of ± 3.0%. The term "about" may denote variability of ± 2.9%. The term "about" may denote variability of ± 2.8%. The term "about" may denote variability of ± 2.7%. The term "about" may denote variability of ± 2.6%. The term "about" may denote variability of ± 2.5%. The term "about" may denote variability of ± 2.4%. The term "about" may denote variability of ± 2.3%. The term "about" may denote variability of ± 2.2%. The term "about" may denote variability of ± 2.1%. The term "about" may denote variability of ± 2.0%. The term "about" may denote variability of ± 1.9%. The term "about" may denote variability of ± 1.8%. The term "about" may denote variability of ± 1.7%. The term "about" may denote variability of ± 1.6%. The term "about" may denote variability of ± 1.5%. The term "about" may denote variability of ± 1.4%. The term "about" may denote variability of ± 1.3%. The term "about" may denote variability of ± 1.2%. The term "about" may denote variability of ± 1.1%. The term "about" may denote variability of ± 1.0%. The term "about" may denote variability of ± 0.9%. The term "about" may denote variability of ± 0.8%. The term "about" may denote variability of ± 0.7%. The term "about" may denote variability of ± 0.6%. The term "about" may denote variability of ± 0.5%. The term "about" may denote variability of ± 0.4%. The term "about" may denote variability of ± 0.3%. The term "about" may denote variability of ± 0.2%. The term "about" may denote variability of 0.1%. The term "about", in reference to the particular recited value, may denote that exact particular value itself, irrespective of any explicit mention that this exact particular value is included; even in the absence of an explicit indication that the term "about" includes the particular exact recited value, this exact particular value is still included in the range of variation created by the term "about", and is therefore disclosed. Unless stated otherwise, if the term "about" is recited before the first endpoint of a numerical range, this term refers to both the first endpoint of the range and the second endpoint of the range. For instance, a recited range of "about X to Y" should be read as "about X to about Y".

In the present application, amounts are often provided in "% by weight". It is to be understood herein that "% by weight" refers to the weight percent of a particular ingredient or ingredients in respect of the total weight of the composition.

As the skilled person will know, a trivial name for *Olea europea* is "olive", a trivial name for *Persea gratissima* is "avocado", a trivial name for *Calendula offinalis* is "marigold" and a trivial name for *Zingiber officinale* is "ginger".

The present invention relates to a composition comprising *Persea gratissima* oil, *Olea europaea* oil, *Calendula officinalis* extract and *Zingiber officinale* oil.

In an embodiment in optional combination with any of the above and below aspects or embodiments, the *Olea europaea* oil is *Olea europeaea* fruit oil, and/or the *Calendula officinalis* extract is *Calendula officinalis* flower extract, and/or the *Zingiber officinale* oil is *Zingiber officinale* root oil.

In a more preferred embodiment in optional combination with any of the above and below aspects or embodiments, the *Olea europaea* oil is *Olea europaea* fruit oil, the *Calendula officinalis* extract is *Calendula officinalis* flower extract and the *Zingiber officinale* oil is *Zingiber officinale* root oil.

In a preferred embodiment in optional combination with any of the above or below aspects or embodiments, the composition essentially consists of *Persea gratissima* oil, *Olea europaea* oil, *Calendula officinalis* extract and *Zingiber officinale* oil.

In the context of the present invention "essentially consisting of" is meant to exclude the presence of other ingredients except possibly in trace amounts, e.g. amounts near the limit of detection for such ingredients. In its furthest limit, in a preferred embodiment, this term is to be interpreted as "consisting entirely of", that is only the recited ingredients are present and nothing else.

As used herein, a substance which is "solid" means that the substance can substantially sustain its physical shape when unsupported by external means, and retains this characteristic at room temperature and up to a temperature of at least 45°C.

The mixture of (fruit and root) oils and calendula (flower) extract confers a naturally pleasant scent on the composition. Thus, the composition can ideally be formulated without natural or synthetic fragrances typically used in cosmetic products such as e.g. linalool, limonene or geraniol. In a preferred embodiment, the present composition is formulated without any natural or synthetic fragrances.

Butylated hydroxytoluene (BHT), also known as dibutylhydroxytoluene, is a lipophilic organic compound with antioxidant properties. Due to these properties, BHT is added in numerous consumer products in the food and cosmetic industry to prevent or slow down changes of products by atmospheric oxygen. However, like many closely related phenol antioxidants, BHT has a low, albeit acute toxicity. In a preferred embodiment, the present composition is formulated without BHT.

Solvents such as water or diluents are not required in the present composition. In a preferred embodiment, the present composition is therefore formulated without water. In such a preferred embodiment, the composition exists as a single oily phase, an aqueous phase being absent. Consequently, the addition of surfactants is not required and the present composition is also preferably formulated without a surfactant, e.g. a polyoxyalkylene carboxylate surfactant.

In an embodiment in optional combination with any of the above or below aspects or embodiments, the composition of the present invention is formulated without additives such as e.g. preservatives, fragrances, emulsifying agents or surfactants.

In a preferred embodiment in optional combination with any of the above or below aspects or embodiments, the inventive composition is formulated without a vitamin such as vitamin B, vitamin D or a tocopherol (e.g. vitamin E or tocopherol acetate), or vitamin A.

In a preferred embodiment in optional combination with any of the above or below aspects or embodiments, the inventive composition is formulated without a propellant, e.g. a hydrocarbon propellant, a gas propellant, in particular a liquefied gas propellant.

In a preferred embodiment in optional combination with any of the above or below aspects or embodiments, the inventive composition is formulated without a foaming agent.

In a preferred embodiment in optional combination with any of the above or below aspects or embodiments, the inventive composition is formulated without palm oil, coconut oil, primrose oil, flax oil, less than 2 wt% Olea europaea (fruit) oil, or pumpkin oil.

In a preferred embodiment in optional combination with any of the above or below aspects or embodiments, the inventive composition is formulated without a paraben.

In a preferred embodiment in optional combination with any of the above or below aspects or embodiments, the inventive composition is formulated without alcohol, e.g. cetyl alcohol, stearyl alcohol, a C₁-C₆ alcohol.

In a preferred embodiment in optional combination with any of the above or below aspects or embodiments, the inventive composition is formulated without an antibiotic, in particular a minocycline antibiotic or a tetracycline antibiotic.

In a preferred embodiment in optional combination with any of the above or below aspects or embodiments, the inventive composition includes only ingredients of vegetable origin. That is, the inventive composition is formulated without any ingredient of animal origin, e.g. lanolin, a birdfeather oil, propolis or beeswax.

In a preferred embodiment in optional combination with any of the above or below aspects or embodiments, the inventive composition is formulated without any solid substance, e.g. a solid particle, a nanoparticle, an absorbent material, a fabric, a clay (e.g. a bentonite such as e.g. quaternium-18 bentonite, a hectorite or organophilic clay (e.g. stearalkonium hectorite, quaternium-18 hectorite), a silica (e.g. magnesium aluminum silicate) or a cloth.

In a preferred embodiment in optional combination with any of the above or below aspects or embodiments, the inventive composition is formulated without any inclusion of gas, e.g a foam.

In a preferred embodiment in optional combination with any of the above or below aspects or embodiments, the inventive composition is formulated without any flower wax of jasmine, lavender, rose, raspberry or violet.

In a preferred embodiment in optional combination with any of the above or below aspects or embodiments, the inventive composition is formulated without (hydrogenated) jojoba (seed) oil, meadowfoam (seed) oil, fennel (seed) oil, flax (seed) oil, almond oil, sesame oil, vine (fruit) oil, grape (seed) oil, oregano oil, apricot oil, fish oil, geranium oil, ylang ylang oil, soy oil, soy isoflavone-3, oyster oil, linseed oil, white leaf oil, safflower (seed) oil, sunflower (seed) oil, macadamia nut oil, castor seed oil, peony seed oil, grapefruit seed oil, apple seed oil, pomegranate seed oil, watermelon seed oil, Cucurbita pepo seed oil, rapeseed oil, sesame seed oil, white chia seed oil, zucchini seed oil, walnut seed oil, baobab seed oil, bitter oil seed oil, *Borago Officinalis* seed oil, *Sclerocarya birrea* seed oil, cotton seed oil, tea seed oil, COIX LACRYMA-JOBI MA-YUEN seed oil, papaya seed oil, raspberry seed oil, monkey bread tree seed oil, bitter oil tree seed oil, moringa seed oil, American pecan seed oil, perilla seed oil, Babassu seed oil, white lupin seed oil, vanillin, lavender oil, Melaleuca alterniflora oil, cephalaria oil, chamomile oil, neem oil, citrus oil, orange oil, frankincense oil, cannabis (seed) oil, or sunflower (seed) wax.

In a preferred embodiment in optional combination with any of the above or below aspects or embodiments, the inventive composition is formulated without a fruit extract (e.g. mango fruit extract), coconut fruit extract, dandelion (root) extract, Matricaria leaf extract, Glycyrrhiza glabra root extract, Centella asiatica extract, Rosemary leaf extract, Scutellaria root extract, tea extract, Polygonum cuspidatum root extract, swertia chirata extract, turmeric, orange extract, frankincense extract, cannabis (seed) extract, or rose flower extract.

In a preferred embodiment in optional combination with any of the above or below aspects or embodiments, the inventive composition is formulated without bis-abolol, calamine or zinc oxide.

In a preferred embodiment in optional combination with any of the above or below aspects or embodiments, the inventive composition is formulated without a solid phase.

In a preferred embodiment in optional combination with any of the above or below aspects or embodiments, the inventive composition is formulated without 90 or more essential oils.

In a preferred embodiment in optional combination with any of the above or below aspects or embodiments, the inventive composition is formulated without a surfactant, in particular an anionic surfactant, a nonionic surfactant, a zwitterionic surfactant, an amphoteric surfactant, a biosurfactant (e.g. a monoramnolipid, a dirramnolipid, a soforolipid, a mannosileritritol lipid, a celubiose, a xylolipid, a trehalipid, a lipopeptide, a glycoside or a rhamnose), a polyoxyalkylene carboxylate surfactant, or a tenside.

In a preferred embodiment in optional combination with any of the above or below aspects or embodiments, the inventive composition is formulated without (hydrogenated) castor oil.

In a preferred embodiment in optional combination with any of the above or below aspects or embodiments, the inventive composition is formulated without petrolatum or a mineral oil.

In a preferred embodiment in optional combination with any of the above or below aspects or embodiments, the inventive composition is formulated without rice bran wax.

In a preferred embodiment in optional combination with any of the above or below aspects or embodiments, the inventive composition is formulated without (hydrogenated) phosphatidylcholine.

In a preferred embodiment in optional combination with any of the above or below aspects or embodiments, the inventive composition is formulated without cyclopentasiloxane, polyethylene glycol (PEG), polypropylene glycol (PPG), PEG/PPG, in particular PEG/PPG 18/18, or dimethicone.

In a preferred embodiment in optional combination with any of the above or below aspects or embodiments, the inventive composition is formulated without cinnamon bark (extract).

In a preferred embodiment in optional combination with any of the above or below aspects or embodiments, the inventive composition is formulated without tea residues.

In a preferred embodiment in optional combination with any of the above or below aspects or embodiments, the inventive composition is formulated without polyvinyl alcohol (PVA).

In a preferred embodiment in optional combination with any of the above or below aspects or embodiments, the inventive composition is formulated without any saponin, e.g. a saponin of *Sapindus mukorossi.*

In a preferred embodiment in optional combination with any of the above or below aspects or embodiments, the inventive composition is formulated without a substance, in particular an oil, which has been processed in such a way to convey one or more aromatic substances to the produced substance, in particular to the produced oil.

In a preferred embodiment in optional combination with any of the above or below aspects or embodiments, the inventive composition is formulated without a stearate, (e.g. glycerol stearate, PEG-stearate, (e.g. PEG-100 stearate), sucrose distearate), PEG-7 olive oil carboxylate, or glyceryl oleate.

In a preferred embodiment in optional combination with any of the above or below aspects or embodiments, the inventive composition is formulated without propanediol, glycerin (glycerine, glycerol), a triglyceride (e.g. a caprylic or capric triglyceride), a biosaccharide, dimethyl isosorbide, ethyoxy diglycerol, or a polycorbate (e.g. polysorbate 20).

In a preferred embodiment in optional combination with any of the above or below aspects or embodiments, the inventive composition is formulated without a moisturizer, e.g. a humectant.

In a preferred embodiment in optional combination with any of the above or below aspects or embodiments, the inventive composition is formulated without 25-40 wt% vegetable wax.

In a preferred embodiment in optional combination with any of the above or below aspects or embodiments, the inventive composition is formulated without a polyphenol or 2-methyloxolane.

In a preferred embodiment in optional combination with any of the above or below aspects or embodiments, the inventive composition is formulated without olive leaf extract.

In a preferred embodiment in optional combination with any of the above or below aspects or embodiments, the inventive composition is formulated without a lignin.

In a preferred embodiment in optional combination with any of the above or below aspects or embodiments, the inventive composition is formulated without an alkyl benzoate (e.g. an C₁₂-C₁₅ alkyl benzoate), isopropyl myristrate, isopropyl palmitate, octyldodecanol, decyl oleate, a cocoglyceride, ethylhexyl stearate, ceteraryl isononanoate, cetearyl ethyhexanonate, decyl cocoate, cetyl dimethicone, ethylhexyl palmitate, PPG-11 stearyl ether, PPG-15 stearyl ether, or PPG-14 butyl ether.

In a preferred embodiment in optional combination with any of the above or below aspects or embodiments, the inventive composition is formulated without a hydrocarbon, a sterol, a tocotrienol, a carotenoid pigment or a xanthophyll.

In a preferred embodiment in optional combination with any of the above or below aspects or embodiments, the inventive composition is formulated without an acid, e.g. a fruit acid, a linoleic acid (e.g. alpha-linoleic acid, gamma-linoleic acid), docosahexanioc acid, eicosapentanoic acid, an omega fatty acid (e.g. an omega-3 fatty acid or an omega-6 fatty acid), a sialic acid or a benzoic acid (e.g. hydroxyphenyl propionamide benzoic acid).

In a preferred embodiment in optional combination with any of the above or below aspects or embodiments, the inventive composition is formulated without dye or colorant. In a preferred embodiment in optional combination with any of the above or below aspects or embodiments, the inventive composition is formulated without an antioxidant.

In a preferred embodiment in optional combination with any of the above or below aspects or embodiments, the inventive composition is formulated without a panthenol (e.g. D-panthenol), a lauric acid (e.g. lauric acid monoglyceride), hyaluronic acid or a salt thereof such as for example sodium hyaluronate, a phospholipid.

In a preferred embodiment in optional combination with any of the above or below aspects or embodiments, the inventive composition is formulated without a sterol, e.g. a phytosterol.

In a preferred embodiment in optional combination with any of the above or below aspects or embodiments, the inventive composition is formulated without an emulsifier, e.g. a lecithin.

In a preferred embodiment in optional combination with any of the above or below aspects or embodiments, the inventive composition is formulated without a skin exfoliative or a skin polishing material.

In a preferred embodiment in optional combination with any of the above or below aspects or embodiments, the inventive composition is formulated without a solvent.

In a preferred embodiment in optional combination with any of the above or below aspects or embodiments, the inventive composition is formulated without a peroxide.

In a preferred embodiment in optional combination with any of the above or below aspects or embodiments, the inventive composition is formulated without an alkyl ketal ester, methyl levulinate propylene glycol ketal, or ethyl levulinate propylene glycol ketyl.

In a preferred embodiment in optional combination with any of the above or below aspects or embodiments, the inventive composition is formulated without vegetable butter.

In a preferred embodiment in optional combination with any of the above or below aspects or embodiments, the inventive composition is formulated without squalene. In a preferred embodiment in optional combination with any of the above or below aspects or embodiments, the inventive composition is formulated without shea butter.

In a preferred embodiment in optional combination with any of the above or below aspects or embodiments, the inventive composition is formulated without glycereth-26.

In a preferred embodiment in optional combination with any of the above or below aspects or embodiments, the inventive composition is formulated without bis-albolol.

In a preferred embodiment in optional combination with any of the above or below aspects or embodiments, the inventive composition is formulated without *Calophyllum inophyllum, Citrus aurantium, Eucalyptus globulus, Eugenia caryophyllata, Foeniculum vulgare, Helichrysum angustifolia, Juniperus virginiana, Lavendula officinalis, Myristica fragrans, Ocimum basilicum, Pinus sylvestris, Piper nigrum, Rosemarinus officinalis, Salvia officinalis lamiacae* or *Salvia sclarea.*

In a preferred embodiment in optional combination with any of the above or below aspects or embodiments, the inventive composition is formulated without Allium spp. extract, Aloe spp. extract, Cinnamomum spp. extract, Curcuma spp. extract, Lavandula spp. extract, Melaleuca spp. extract, Plantago extract, Salvia officinalis extract, Salvia miltiorrhiza extract, or Thymus spp. extract.

In a preferred embodiment in optional combination with any of the above or below aspects or embodiments, the inventive composition is formulated without a ketal.

In a preferred embodiment in optional combination with any of the above or below aspects or embodiments, the inventive composition is formulated without a cyclodextrin.

In a preferred embodiment in optional combination with any of the above or below aspects or embodiments, the inventive composition is formulated without sodium acrylate, sodium acryloyl dimethyl taurate, or a sodium acrylate/sodium acryloyl dimethyl taurate copolymer.

In addition, the ingredients in the inventive composition are advantageously - and preferably - non-toxic and certified organic. The natural, organic nature of the inventive composition has the effect that the breast-feeding mother need not be concerned about possible ingestion of the inventive composition by the infant. Following application of the inventive composition to sore breasts, e.g. for a soothing self-massage after a breast-feeding or pumping session, the inventive composition can therefore be left on the skin, i.e. does not need to be washed off, prior to subsequent breast-feeding or pumping sessions to prevent unwanted ingestion by the breast-feeding infant, or by the infant who will ingest pumped breast milk. The inventive composition is thus advantageously a "leave-on product".

This is advantageous for a number of reasons. For instance, the ability to leave the inventive composition on sore breasts means that the breast-feeding or breast milk-pumping mother need not plan in time to wash off a previous application of the inventive composition prior to any following breast-feeding or -pumping session. The breast-feeding or breast milk-pumping mother thus gains additional time to accrue the benefit of relief from a previous application of the inventive composition, while also maximizing time between breast-feeding or pumping sessions for other activities such as e.g. regenerative sleep, which is an especially precious commodity in the daily routine of any new mother. Further, the ability to dispense with intermittent washing cycles also means that the breast-feeding or breast milk-pumping mother can avoid further insulting already-sore breasts by the irritating mechanical process of washing and drying.

Of course, the inclusion of additives need not automatically disqualify such a composition from classification as a "leave-on product", but the lack of any need to consider and additionally insure the safety and e.g. organic certification of any such additives or preservatives constitutes a particular advantage of the composition of the present application.

Lacking any additives such as e.g. preservatives, fragrances or surfactants, the composition of the present invention has the additional advantage of being hypoallergenic and can thus be used by adults and children, including babies, to relieve sore or dry skin on, e.g. promoting massaging of any part of the body. In an embodiment of the present invention, it is therefore contemplated that the inventive composition can be used cosmetically by subjects having normal to dry and/or sore skin anywhere on the body. In standing with the above, however, the composition of the present invention is particularly useful for breastfeeding women who often suffer from sore breasts, nipples and/or dry skin in the nipple region, and as mentioned above is particularly well-suited for direct application to sore breasts and/or dry nipples and the surrounding nipple region to relieve such soreness and dryness between breastfeeding sessions by allowing a soothing massage of the region. As mentioned above, the non-toxic nature of the inventive composition, and in particular its organic certification, has the advantage that the composition can be safely used as a leave-on product for the warming relief of sore breasts nipples and dry skin, with the associated advantages described above.

In an embodiment in optional combination with any of the above or below aspects or embodiments, *Persea gratissima* oil is present within the composition in an amount of about 80% to about 100% by weight, such as about 80% to about 99%, about 81% to about 100%, about 82% to about 100%, about 83% to about 100%, about 84% to about 100%, about 81% to about 99%, about 82% to about 99%, about 83% to about 99%, or about 84% to about 99% by weight.

In preferred embodiment in optional combination with any of the above or below aspects or embodiments, *Persea gratissima* oil is present within the composition in an amount of about 85% to about 99% by weight, such as about 85% to about 98%, about 85% to about 97%, about 86% to about 99%, about 86% to about 98%, about 86% to about 97%, about 87% to about 99%, about 87% to about 98%, about 87% to about 97%, about 88% to about 99%, about 88% to about 98%, about 88% to about 97%, about 89% to about 99%, about 89% to about 98%, or about 89% to about 97% by weight.

In a more preferred embodiment in optional combination with any of the above or below aspects or embodiments, *Persea gratissima* oil is present within the composition in an amount of about 90% to about 96% by weight, such as about 90% to about 95%, about 90% to about 94%, about 90% to about 93%, about 91 % to about 96%, about 91% to about 95%, about 91% to about 94%, or about 91% to about 93% by weight.

Even more preferably in optional combination with any of the above or below aspects or embodiments, *Persea gratissima* oil is present within the composition in an amount of about 91% to about 92% by weight, such as about 91.5% to about 92% or about 91% to about 91.9% by weight, most preferably in an amount of about 91.9% by weight.

In an embodiment in optional combination with any of the above or below aspects or embodiments, *Olea europaea* (fruit) oil is present within the composition in an amount of about 1% to about 20% by weight, such as about 1% to about 19%, about 1% to about 18%, about 1% to about 17%, about 1% to about 16%, about 2% to about 20%, about 2% to about 19%, about 2% to about 18%, about 2% to about 17%, or about 2% to about 16% by weight.

In a preferred embodiment in optional combination with any of the above or below aspects or embodiments, *Olea europaea* (fruit) oil is present within the composition in an amount of about 3% to about 15% by weight, such as about 3% to about 14%, about 3% to about 13%, about 3% to about 12%, about 3% to about 11%, about 4% to about 15%, about 4% to about 14%, about 4% to about 13%, about 4% to about 12%, or about 4% to about 11% by weight.

In a more preferred embodiment in optional combination with any of the above or below aspects or embodiments, *Olea europaea* (fruit) oil is present within the composition in an amount of about 5% to about 10% by weight, such as about 5% to about 9%, about 5% to about 8%, about 6% to about 10%, about 6% to about 9%, about 6% to about 8%, about 6,5% to about 10%, about 6.5% to about 9%, or about 6.5% to about 8% by weight, most preferably in an amount of about 7% by weight.

In an embodiment in optional combination with any of the above or below aspects or embodiments, *Calendula officinalis* (flower) extract is present within the composition in an amount of about 0.01% to about 5% by weight, such as about 0.01% to about 4.5%, about 0.01% to about 4%, about 0.01% to about 3.5%, about 0.03% to about 5%, about 0.03% to about 4.5%, about 0.03% to about 4%, about 0.03% to about 3.5%, about 0.05% to about 5%, about 0.05% to about 4.5%, about 0.05% to about 4%, about 0.05% to about 3.5%, about 0.7% to about 5%, about 0.07% to about 4.5%, about 0.07% to about 4%, about 0.07% to about 3.5%, about 0.09% to about 5%, about 0.09% to about 4.5%, about 0.09% to about 4%, or about 0.09% to about 3.5% by weight.

In preferred embodiment in optional combination with any of the above or below aspects or embodiments, *Calendula officinalis* (flower) extract is present within the composition in an amount of about 0.1% to about 3% by weight, such as 0.1% to about 2.8%, about 0.2% to about 3%, about 0.2% to about 2.8%, about 0.3% to about 3%, about 0.3% to about 2.8%, about 0.4% to about 3%, or about 0.4% to about 2.8% by weight

In a more preferred embodiment in optional combination with any of the above or below aspects or embodiments, *Calendula officinalis* (flower) extract is present within the composition in an amount of about 0.5% to about 2.5% by weight such as about 0.5% to about 2.3%, about 0.5% to about 2.1%, about 0.5% to about 1.9%, about 0.5% to about 1.7%, about 0.5% to about 1.5%, about 0.5% to about 1.3%, about 0.7% to about 2.5%, about 0.7% to about 2.3%, about 0.7% to about 2.1%, about 0.7% to about 1.9%, about 0.7% to about 1.7%, about 0.7% to about 1.5%, about 0.7% to about 1.3%, about 0.9% to about 2.5%, about 0.9% to about 2.3%, about 0.9% to about 2.1%, about 0.9% to about 1.9%, about 0.9% to about 1.7%, about 0.9% to about 1.5%, or about 0.9% to about 1.3% by weight. Most preferably, *Calendula officinalis* extract is present within the composition in an amount of about 1.0% by weight.

In an embodiment in optional combination with any of the above or below aspects or embodiments, *Zingiber officinale* (root) oil is present within the composition in an amount of about 0.001% to about 2% by weight, such as about 0.001% to about 1.7%, about 0.001% to about 1.5%, about 0.001% to about 1.3%, about 0.003% to about 2% by weight, 0.003% to about 1.7%, about 0.003% to about 1.5%, about 0.003% to about 1.3%, about 0.005% to about 2% by weight, 0.005% to about 1.7%, about 0.005% to about 1.5%, about 0.005% to about 1.3%, about 0.007% to about 2% by weight, 0.007% to about 1.7%, about 0.007% to about 1.5%, about 0.007% to about 1.3%, about 0.009% to about 2% by weight, 0.009% to about 1.7%, about 0.009% to about 1.5%, or about 0.009% to about 1.3% by weight.

In preferred embodiment in optional combination with any of the above or below aspects or embodiments, *Zingiber officinale* (root) oil is present within the composition in an amount of about 0.01 to about 1% by weight, such as about 0.01% to about 0.9%, about 0.01% to about 0.8%, about 0.01% to about 0.7%, about 0.01% to about 0.6%, about 0.02% to about 1%, about 0.02% to about 0.9%, about 0.02% to about 0.8%, about 0.02% to about 0.7%, about 0.02% to about 0.6%, about 0.03% to about 1%, about 0.03% to about 0.9%, about 0.03% to about 0.8%, about 0.03% to about 0.7%, about 0.03% to about 0.6%, about 0.04% to about 1%, about 0.04% to about 0.9%, about 0.04% to about 0.8%, about 0.04% to about 0.7%, or about 0.04% to about 0.6% by weight.

In a more preferred embodiment in optional combination with any of the above or below aspects or embodiments, *Zingiber officinale* (root) oil is present within the composition in an amount of about 0.05% to about 0.5% by weight, such as about 0.05% to about 0.45%, about 0.05% to about 0.4%, about 0.05% to about 0.35%, about 0.05% to about 0.3%, about 0.05% to about 0.25%, about 0.05% to about 0.2%, 0.07% to about 0.5% by weight, about 0.07% to about 0.45%, about 0.07% to about 0.4%, about 0.07% to about 0.35%, about 0.07% to about 0.3%, about 0.07% to about 0.25%, about 0.07% to about 0.2%, 0.09% to about 0.5% by weight, about 0.09% to about 0.45%, about 0.09% to about 0.4%, about 0.09% to about 0.35%, about 0.09% to about 0.3%, about 0.09% to about 0.25%, or about 0.09% to about 0.2% by weight, even more preferred about 0.095% to about 0.15% by weight. Most preferably, *Zingiber officinale* (root) oil is present within the composition in an amount of about 0.1% by weight.

In an embodiment in optional combination with any of the above or below aspects or embodiments, the composition comprises about 80% to about 100% by weight of *Persea gratissima* oil, about 1% to about 20% by weight of *Olea europaea* (fruit) oil, about 0.01% to about 5% by weight of *Calendula officinalis* (flower) extract and about 0.001% to about 2% by weight of *Zingiber officinale* (root) oil.

In an embodiment in optional combination with any of the above or below aspects or embodiments, the composition comprises about 85% to about 99% by weight of *Persea gratissima* oil, about 3% to about 15% by weight of *Olea europaea* (fruit) oil, about 0.1% to about 3% by weight of *Calendula officinalis* (flower) extract and about 0.01% to about 1% by weight of *Zingiber officinale* (root) oil.

In a more preferred embodiment in optional combination with any of the above or below aspects or embodiments, the composition comprises about 90% to about 96% by weight of *Persea gratissima* oil, about 5% to about 10% by weight of *Olea europaea* (fruit) oil, about 0.5% to about 2.5% by weight of *Calendula officinalis* (flower) extract and about 0.05% to about 0.5% by weight of *Zingiber officinale* (root) oil.

In a most preferred embodiment in optional combination with any of the above or below aspects or embodiments, the composition of the present invention consists of 91.9% by weight of *Persea gratissima* oil, about 7% by weight of *Olea europaea* (fruit) oil, 1% by weight of *Calendula officinalis* (flower) extract and about 0.1% by weight of *Zingiber officinale* (root) oil.

In a most preferred embodiment in optional combination with any of the above or below aspects or embodiments, the composition of the present invention consists of 91.9% by weight of *Persea gratissima* oil, about 7% by weight of *Olea europaea* fruit oil, 1 % by weight of *Calendula officinalis* flower extract and 0.1% by weight of *Zingiber officinale* root oil.

In a further most preferred embodiment in optional combination with any of the above or below aspects or embodiments, the composition of the present invention consists of 91.90000% by weight of *Persea gratissima* oil, about 7.00000% by weight of *Olea europaea* fruit oil, 1 .00000% by weight of *Calendula officinalis* flower extract and 0.10000% by weight of *Zingiber officinale* root oil.

*Persea gratissima* (avocado) is a medium-sized, evergreen tree in the laurel family (Lauraceae). Synonyms are *Persea americana, Laurus persea, Persea drymifolia* and *Persea nubigena.* In the United Kingdom, the term "avocado pear" is sometimes used while it is known as "butter fruit" in parts of India and Hong Kong.

The avocado fruit is a single-seeded berry. Inside, in addition to the pulp, it has a large seed that accounts for about 13-18% of the total fruit. Avocado oil can be obtained by mechanically extracting both the seed and the pulp. For preparing the *Persea gratissima* oil in the inventive composition of the present invention, only the fleshy pulp surrounding the avocado seed is pressed. Preferably, the fruits are peeled before pressing.

Avocado oil is usually extracted from the avocado pulp by cold pressing or expeller pressing. Extraction may also be assisted by enzymes or the oil may be hot pressed and extracted using solvents. According to a preferred embodiment of the present invention, the avocado oil is extracted from the pulp by expeller pressing to preserve the nutrients contained in the avocado pulp.

An expeller press is a screw-type machine which presses oil through a caged barrel-like-cavity, using friction and continuous pressure. The screw drives forward to literally squeeze the oil from the compressed seeds. There isn't any added heat in this process, but the pressure and friction involved in the pressing process creates heat in the range of 60-99°C. *Persea gratissima* oil is obtained according to processes known to the skilled person.

In a preferred embodiment of the present invention in optional combination with any of the above or below aspects or embodiments, the *Persea gratissima* oil may be obtained by expeller pressing avocado pulp according to processes known to the skilled person. This involves separating the fruits from unwanted components such as chaff or dust and then drying the fruits. After removal of the pit, the resulting mass is subjected to expeller pressing. The crude oil is filtrated followed by a refining process.

In a preferred embodiment in optional combination with any of the above or below aspects or embodiments, the inventive composition is formulated without hydrogenated *Persea gratissima* oil.

The *Persea gratissima* oil in the inventive composition may alternatively be obtained commercially, for example from the supplier O&3, product code K0821.

*Olea europaea* (olive) is a medium-sized tree, from the olive tree genus (Olea), which belongs to the olive family (Oleaceae). It is widely cultivated in the mediterranean area for its fruits. About 90% of the annual harvest of olives is pressed to olive oil, also known as Oleaster oil.

The *Olea europaea* (fruit) oil used in the present composition may be obtained by processing the whole fruit including the pulp and kernel. In a preferred embodiment in optional combination with any of the above or below embodiments, the kernel is separated and only the pulp is used for preparing the olive fruit oil. The (fruit) oil can be produced by grinding olives and extracting the oil by mechanical or chemical means. The olive (fruit) oil in the present invention may be prepared by employing a mechanical process as is known in the art.

At present, there are two main mechanical processes for extracting oil from olives.

The first process, more conventional and discontinuous, includes a first step of crushing the olives with granite mill stones, a second step of kneading, followed in a third step by the extraction of the oil by means of a pressure, and a subsequent centrifugal separation of the light solids and aqueous phase from the oil.

The second process, more modern, uses continuous mills of various types, such as hammer, blade or disk mill, to crush the olives. The resulting pulpy mass is then stirred in a kneader to have the fine oil droplets coalesce. Finally, a centrifugate separates the light solids, the aqueous phase and the oil. Alternatively, the pulpy mass is expeller pressed.

Preferably, the olive (fruit) oil may be filtered after centrifugation to eliminate remaining solid particles and to enhance shelf life of the product.

The *Olea europaea* (fruit) oil in the inventive composition may alternatively be obtained commercially, for example from the supplier O&3, product code K0936.

In a preferred embodiment of the present invention in optional combination with any of the above or below aspects or embodiments, the *Olea europaea* fruit oil may be obtained by expeller pressing olive fruits according to processes known to the skilled person. This involves separating the fruits from unwanted components such as chaff or dust and removing the kernel. The olive fruits are then subjected to expeller pressing. The crude oil is filtrated followed by a deodorisation process.

In a preferred an embodiment of the present invention in optional combination with any of the above or below aspects or embodiments, the inventive composition is formulated without hydrogenated *Olea europaea* (fruit) oil.

*Calendula officinalis,* also known as pot marigold, common marigold, ruddles, Mary's gold or Scotch marigold, is a flowering plant in the daisy family Asteraceae. Because the florets of *Calendula officinalis* are edible, they are often used to add color to salads or added to dishes as a garnish and in lieu of saffron. The leaves are edible but are often not palatable. The plant, in particular the petal, is also used to make tea.

In a preferred embodiment of the present invention in optional combination with any of the above or below aspects or embodiments, an extract of the fresh plant material from *Calendula officinalis* may be used. The extraction may be carried out by infusing the plant material in vegetables oils, such as olive oil in a herb: oil ratio from 1:5 to 1:25. During extraction, constant contact between the components may be ensured by continuous stirring. The extraction may be followed by a filtration step.

Typically, the *Calendula officinalis* extract is made solely of the flower. In the context of the present invention, the term "flower" includes the receptacle, the sepals, the petals, the stamina and the pistil(s). In a preferred embodiment of the present invention in optional combination with any of the above or below aspects or embodiments, the *Calendula officinalis* (flower) extract used in the inventive composition is an extract of the flower(s) with olive oil. That is, the *Calendula officinalis* (flower) extract is preferably not obtained from extracting the seeds of *Calendula officinalis.* The *Calendula officinalis* (flower) extract may be further preferably formulated without unsaponifiable material. The *Calendula officinalis* (flower) extract used in the composition of the invention may be prepared by methods known to the skilled person.

The *Calendula officinalis* (flower) (extract) in the inventive composition may alternatively be obtained commercially, for example from the supplier O&3, product code K0970.

*Zingiber officinale* (ginger) is a flowering plant and belongs to the family Zingiberaceae. It is a herbaceous perennial which grows annual pseudostems (false stems made of the rolled bases of leaves) about one meter tall bearing narrow leaf blades. The inflorescences bear flowers having pale yellow petals with purple edges, and arise directly from the rhizome on separate shoots. Common names of *Zingiber officinale* are common ginger, canton ginger or stem ginger.

*Zingiber officinale* is a common spice used worldwide, whether for meals or as a folk medicine or in cosmetic products. For example, it can be used for a variety of food items such as vegetables, candy, soda, pickles, and alcoholic beverages. Typically, in food items the root and the rhizome are used either in fresh, powdered or pickled form.

In addition to roots, stems and leaves, rhizomes are also part of plants: rhizomes are stems of a plant that store nutrients and thus act as a reserve organ for the plant. In some plants, the stems grow horizontally rather than vertically into branches that end in leaves. Unlike roots, rhizomes have internodes. In addition, rhizomes have leaf scars. From them, in turn, descend downward the actual roots. In the context of the present invention, the term "*Zingiber officinale* root" or "ginger root" is used in its everyday sense as referring to the rhizome of *Zingiber officinalis.*

The cosmetic industry typically applies ginger in the form of an extract or oil, depending on the envisaged use and effect. An extract of the rhizome of *Zingiber officinale* can be obtained by extraction from the rhizome with lipophilic solvents such as ethyl acetate, hexane and aliphatic esters, or by extraction with carbon dioxide under supercritical conditions.

To obtain ginger oil, the tuber is cut into small pieces and worked with a kind of hammer to open the pores. During the following distillation in steam, the essential oil is gradually extracted from the ginger. Then the liquid condenses in a cool tube, where the oil separates from the water. Since the ginger oil still contains about five percent water at this point, a second distillation process must be performed to remove residual water.

Alternatively, the ginger oil can be prepared by cutting the rhizome, pressing or grating the pieces to obtain a pulpy juice and mixing the juice with a vegetable oil including but not limited to sunflower oil or olive oil. Finally, the mixture is filtered.

In a preferred embodiment of the present invention in optional combination with any of the above or below aspects or embodiments, the ginger (root) oil in the inventive composition is prepared from the rhizome of *Zingiber officinalis.* Preferably, the rhizome is used without further processing, namely without peeling the rhizome before cutting. In a further preferred embodiment of the present invention in optional combination with any of the above or below aspects or embodiments, the *Zingiber officinale* oil is obtained via steam distillation as described above.

The *Zingiber officinale* (root) oil in the inventive composition may alternatively be obtained commercially, for example from the supplier The Lebermuth Company, Inc., product number 50-6135-01.

The composition of the present invention may be prepared and applied topically in any suitable form. In a preferred embodiment, the composition is prepared as an oil.

The composition may be applied to any body portion in need of a massage such as cramped muscles of the calf, thigh, hand and so on, where a warming effect is desired and/or where massage may soothe. The composition of the present invention is particularly useful to breastfeeding mothers suffering from a feeling of breast tightness, where the warming effect of the inventive composition and/or a massage may soothe discomfort.

Application of the composition of the present invention can be accomplished by any suitable means, such as rubbing, rolling or massaging. The composition of the present invention is particularly suitable for application during a massage session.

The composition of the present invention has an extremely soothing effect, e.g. when sensations of tightness in the breast occur during pregnancy as well as during breastfeeding after birth. The mild composition is based on avocado oil, which makes the skin supple and smooth, and in combination with a gentle massage promotes the circulation of the tissue of the breast, soothing discomfort. Further, avocado oil does not absorb quickly into the skin, meaning that its lubricating effect an support a longer soothing massage than would be possible with many other oils. Ginger oil provides a pleasant warming sensation and helps the breastfeeding mother to relax. The massage with the inventive composition thus supports a harmonious breastfeeding relationship.

### Examples

The present invention will be illustrated by the following non-limiting examples.

### Example 1

A composition is prepared by thoroughly mixing the ingredients in an amount as shown in Table 1.

**Table 1**

| **Ingredients** | **%w/w in Final Product Formulation** |
|---|---|
| Persea gratissima (avocado) oil | 91.90000 % |
| Olea europaea (olive) fruit oil | 7.00000 % |
| Calendula officinalis flower | 1 .00000 % |
| Zingiber officinale root oil | 0.100000% |
| TOTAL | 100 % |

All ingredients are Cosmos Organic Certified or of food grade.

### Example 2

A composition is prepared by thoroughly mixing the ingredients in an amount as shown in Table 2.

**Table 2**

| **Ingredients** | **Amount in 2 ml (1.84 g) composition [g]** |
|---|---|
| Persea gratissima (avocado) oil | 1.690960 |
| Olea europaea (olive) fruit oil | 0.128800 |
| Calendula officinalis flower | 0.018400 |
| Zingiber officinale root oil | 0.001840 |
| TOTAL | 1.84 |

All ingredients are Cosmos Organic Certified or of food grade.

### Example 3

A composition is prepared by thoroughly mixing the ingredients in an amount as shown in Table 3.

**Table 3**

| **Ingredients** | **Amount in 50 ml (46 g) composition [g]** |
|---|---|
| Persea gratissima (avocado) oil | 42.27400 |
| Olea europaea (olive) fruit oil | 3.22000 |
| Calendula officinalis flower | 0.46000 |
| Zingiber officinale root oil | 0.04600 |
| TOTAL | 46.0000 |

All ingredients are Cosmos Organic Certified or of food grade.

## Claims

1. A composition comprising *Persea gratissima* oil, *Olea europaea* oil, *Calendula officinalis* extract and *Zingiber officinale* oil.

2. The composition according to claim 1, wherein
• the *Olea europaea* oil is *Olea europeaea* fruit oil, and/or
• the *Calendula officinalis* extract is *Calendula officinalis* flower extract, and/or
• the *Zingiber officinale* oil is *Zingiber officinale* root oil,
preferably wherein the *Olea europaea* oil is *Olea europaea* fruit oil, the *Calendula officinalis* extract is *Calendula officinalis* flower extract and the *Zingiber officinale* oil is *Zingiber officinale* root oil.

3. The composition according to claim 1 or 2, wherein the composition comprises about 80% to about 100% by weight, preferably about 85% to about 99% by weight, more preferably about 90% to about 96% by weight, even more preferably about 91% to about 92 % by weight, most preferably about 91.9% by weight of the *Persea gratissima* oil.

4. The composition according to any one of claims 1-3, wherein the composition comprises about 1% to about 20% by weight, preferably about 3% to about 15% by weight, more preferably about 5% to about 10% by weight, most preferably about 7 % by weight of the *Olea europaea* oil.

5. The composition according to any one of claims 1-4, wherein the composition comprises about 0.01% to about 5% by weight, preferably about 0.1% to about 3% by weight, more preferably about 0.5% to about 2.5% by weight, most preferably about 1% by weight of the *Calendula officinalis* extract.

6. The composition according to any one of claims 1-5, wherein the composition comprises about 0.001% to about 2% by weight, preferably about 0.01% to about 1% by weight, more preferably about 0.05% to about 0.5% by weight, even more preferably about 0.095% by weight to about 0.15 % by weight, most preferably about 0.1% by weight of the *Zingiber officinale* oil.

7. The composition according to any one of claims 1-6, wherein the cumulative amount of *Persea gratissima* oil, *Olea europaea* oil, *Calendula officinalis* extract and *Zingiber officinale* oil in the composition is more than about 90% by weight, preferably more than about 95% by weight, more preferably more than about 99% by weight.

8. The composition according to claim 7, wherein the composition essentially consists of *Persea gratissima* oil, *Olea europaea* oil, *Calendula officinalis* extract and *Zingiber officinale* oil.

9. The composition according to any one of the preceding claims, wherein the ingredients are of certified organic origin.

10. The composition according to anyone of the preceding claims, wherein the composition is in the form of an oil.

11. A cosmetic use of the composition according to any one of claims 1-10 for relieving sore breasts.
